# EUROPEAN PATENT SPECIFICATION

(11) **EP 0 610 326 B1**
(45) Date of publication and mention of the grant of the patent: **18.09.2002**
(21) Application number: 92922398.0
(22) Date of filing: 14.10.1992
(51) Int. Cl.: A61B 18/00

(54) **ELECTROSURGICAL CUTTING TOOL**
ELEKTROCHIRURGISCHES SCHNEIDWERKZEUG
INSTRUMENT ELECTROCHIRURGICAL COUPANT

(30) Priority: 01.11.1991 US 786572
(43) Date of publication of application: 17.08.1994
(73) Proprietor: MEDICAL SCIENTIFIC, INC., Taunton, MA 02780 (US)
(72) Inventor: NARDELLA, Paul, C., North Easton, MA 02356 (US)
(74) Representative: Casey, Lindsay Joseph
(86) International application number: US9208776
(87) International publication number: WO93008754

(56) References cited:
- US-A- 1 881 250
- US-A- 3 651 811
- US-A- 3 952 748
- US-A- 4 334 539
- US-A- 4 784 137
- US-A- 4 815 465

## Description

### Background of the Invention

The present invention relates to an electrosurgical tool which is adapted to simultaneously cut, fuse, and cauterize the cut tissue so as to improve hemostasis.

Surgical procedures often require incisions to be made in internal organs, such as the intestine, causing profuse bleeding at the site of the incision. Prompt control or elimination of the bleeding is of paramount importance to the success and safety of the procedure.

Currently known surgical cutting devices utilize different techniques to control or eliminate bleeding. One known device is the Proximate Linear Cutter available from the Ethicon, Inc. of Sommerville, New Jersey. This device is specifically adapted to make an incision in tissue or an organ such as the intestine. The device engages a portion of the tissue or organ between two tyne-like members. To effect cutting, a blade mounted on one of the tynes travels along a predetermined path, thereby making a linear incision through the tissue or organ. Simultaneously, surgical staples are deployed by the cutting device on either side of the incision, resulting in the separation of the organ into two segments, each of which is sealed adjacent to the incision by surgical staples. Despite the use of surgical staples and the precise cutting of the tissue, bleeding is not entirely eliminated and separate cauterization procedures must often be utilized to control or stop bleeding.

Surgical devices also are known which utilize electrical current in the form of radio frequency (RF) energy to cauterize tissue and to prevent or control bleeding. U.S. Patent No. 4,651,734 discloses a surgical scalpel modified to include an electrode. This scalpel has the ability to cut tissue and, when properly positioned, to cauterize tissue following a cutting procedure. Such a surgical tool is useful but does not simultaneously cut and cauterize tissue. The separate cauterization procedure which must be utilized is relatively time consuming and may result in unnecessary bleeding. Moreover, such a scalpel is not well suited to many surgical procedures such as the transection of the intestine.

Accordingly, there is a need for a surgical tool which conveniently and safely enables precise incisions to be made in internal organs, and which simultaneously is able to eliminate essentially all bleeding which results from the incision.

It is thus an object of the invention to provide a surgical tool which has improved cutting capability and which decreases some of the risk associated with surgery by minimizing the amount of bleeding resulting from incisions. Another object is to provide a surgical tool which is adapted to simultaneously cut tissue and to cauterize the cut tissue. A further object is to provide an electrosurgical tool which is specifically adapted to make linear incisions in internal organs and, simultaneously, to fuse the tissue adjacent to the incision in order to eliminate any associated bleeding. Other objects of the invention will be apparent upon reading the disclosure which follows. According to the present invention, there is provided an electrosurgical cutting device in accordance with claim 1.

The present invention comprises an electrosurgical cutting tool which is able to effect a precise incision through tissue, while at the same time ensuring that essentially all of the bleeding which results from the incision is controlled or eliminated. The electrosurgical cutting tool features a housing which includes a handle portion and a cutting template element which is disposed adjacent to the handle portion of the housing. The cutting template preferably includes first and second elongate tyne elements which define a tissue engaging space. A first tyne element includes a retractable cutting blade which is adapted to travel along a linear cutting path defined within the first tyne. The cutting blade is electrically insulated from the remainder of the tool and is in electrical communication with an active electrode which provides a source of electrosurgical energy to the blade. The surgical cutting tool of the invention also includes a mechanism, preferably located on the handle, which controls the movement of the blade along the cutting path.

The electrosurgical cutting tool is a bipolar device. In this device, the active electrode supplies electrical current to the blade, and the return electrode is disposed on a tissue-contacting portion of the second tyne.

The electrosurgical energy provided to the cutting blade, preferably in the form of radio frequency energy, improves the mechanical cutting ability of the blade, and more importantly, facilitates cauterization and/or fusion of the tissue following the incision. It has been found that the use of radio frequency energy in connection with the cutting tool effectively allows the simultaneous cutting of tissue, and cauterizing and fusing of tissue adjacent the incision in order to eliminate virtually all resulting bleeding.

In another embodiment of the invention a plurality of surgical staples may be deployed by the device during a cutting procedure. In this embodiment a surgical staple cartridge is disposed within the first tyne, defining a central longitudinal groove through which the cutting blade is able to travel. The surgical staple cartridge includes a plurality of staples, preferably disposed in dual rows on either side of the longitudinal groove. Upon movement of the blade, a staple ejecting device travels with the blade along the length of the staple cartridge causing the staples to be deployed through the tissue. A staple closing mandrel preferably is disposed in the second tyne to effect closure of the staples. This embodiment is advantageous as it allows the tissue to be cut, and at the same time, enables a row of staples to be deployed adjacent the incision while electrical current is passed through the blade to eliminate bleeding by effecting cauterization and tissue fusion. In some instances it may be desirable to deliver electrosurgical energy through the surgical staples as well as through the blade.

### Brief Description of the Drawings

Figure 1 schematically illustrates the surgical cutting tool of the invention, including a supply source of electrosurgical energy.

Figure 2 is an exploded side view of the electrosurgical cutting tool illustrated in Figure 1.

Figure 3 is a sectional view of the electrosurgical tool of Figure 2 at lines A-A.

Figure 4 is a sectional view of the electrosurgical tool of Figure 2, at lines B-B.

Figure 5 is a sectional view of the electrosurgical tool of Figure 2 at lines B-B in an embodiment which does not include a surgical staple cartridge.

### Detailed Description of the Invention

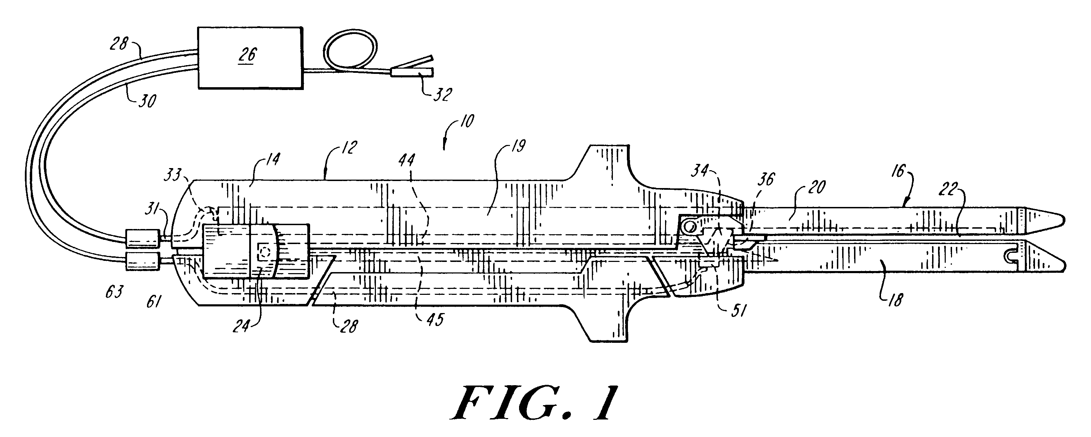

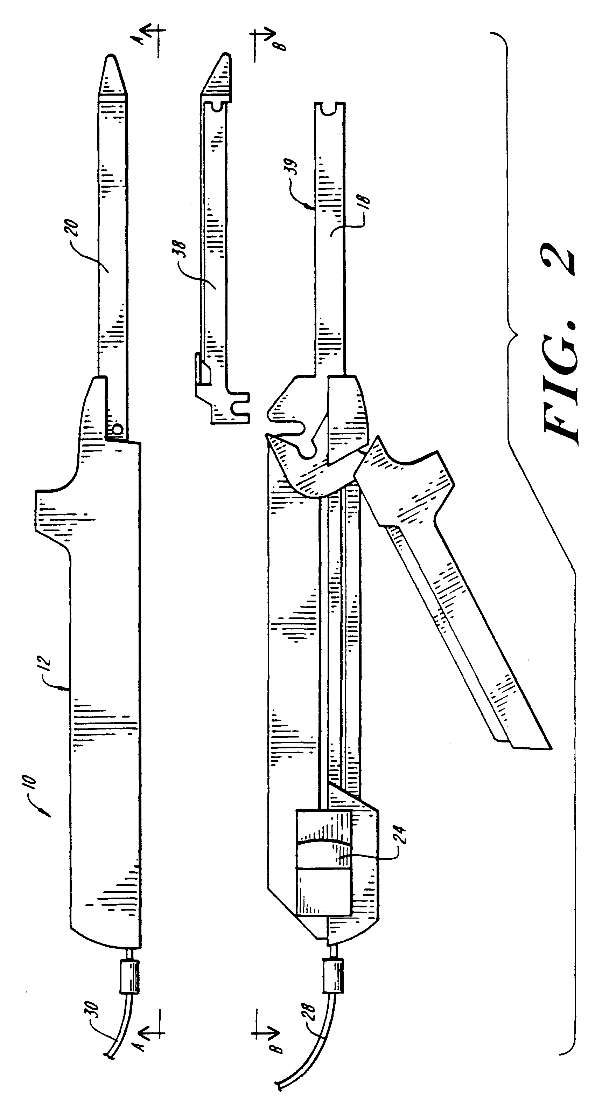

Figures 1 and 2 illustrate one embodiment of the invention in which the-surgical cutting tool 10 comprises a housing 12 including a handle portion 14. Adjacent handle portion 14 is cutting template element 16 which includes a first tyne 18 and a second tyne 20. The two tynes 18, 20 of cutting template element 16 are substantially parallel and define a tissue engaging space 22 into which is inserted the tissue or organ to be incised. In a preferred embodiment, the surgical tool 10 includes a lever 24 which facilitates the movement of a cutting blade 34 along a predetermined path.

Figure 1 further illustrates an electrosurgical generator 26 which serves as an energy source from which electrical current, preferably in the radio frequency range, is communicated to the cutting tool through insulated wire 28. Insulated wire 30 communicates through connector 31 and internal ground wire 33 with a conductive portion of tyne 20 which serves as a ground. A control switch 32, preferably in the form of a foot pedal, may be used to control the power supplied to the cutting tool. Alternatively, a control switch may be disposed on a portion of the cutting tool such as the housing 12.

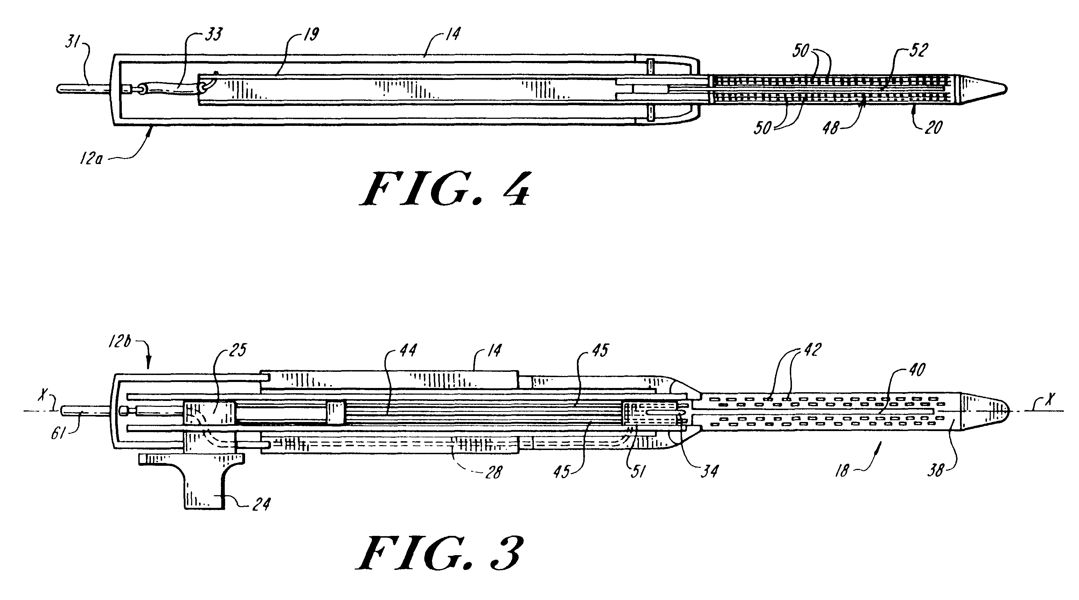

As best shown in Figures 1 and 3, blade 34 can be retracted when not in use. In the retracted position blade 34 is disposed rearward of the first tyne 18 within a forward portion of housing 12. Blade 34 includes a cutting edge 36 disposed at the leading edge of the blade. Further, a blade actuation arm 44 which extends into housing 12 is either attached to or integral with blade 34. The blade 34 is adapted to move along the longitudinal axis x of the tyne 18 upon actuation of lever 24 in order to effect the cutting of tissue.

A surgical staple cartridge 38 may optionally be seated within the first tyne 18, as illustrated in Figures 1 through 3. Cartridge 38 is adapted to securely fit within a channel 39 formed in tyne 18. The staple cartridge 38 includes a central cutting groove 40 through which the cutting blade 34 passes during a cutting procedure. Dual rows of openings 42 through which surgical staples (not shown) emerge straddle either side of groove 40.

As further illustrated in Figures 1 and 3, lever 24 preferably is connected to the blade 34 through an actuation arm 44. Forward movement of lever 24 thus effects movement of the blade 34 causing it to traverse the cutting groove 40. Preferably, a staple ejecting mechanism, such as ejection arms 45, is actuated simultaneous with actuation of the blade. In this way staples are ejected through openings 42 as the blade traverses the groove 40. As shown in the illustrated embodiment lever 24 may be connected to ejection arms 45 such that movement of the lever 24 also controls movement of the ejection arms 45.

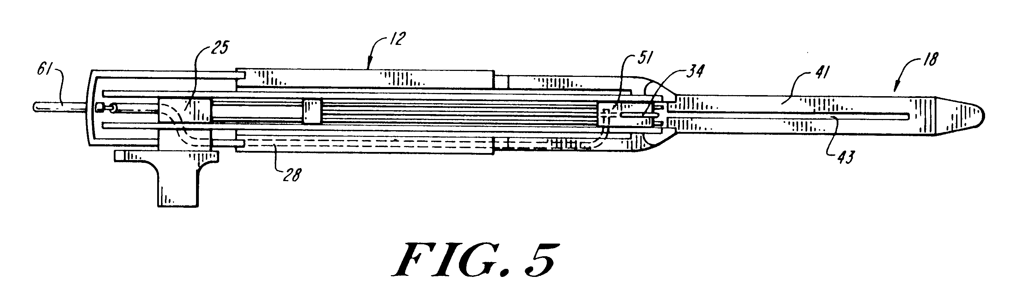

Figure 5 illustrates an embodiment of the invention in which the electrosurgical cutting tool does not utilize surgical staples. In this embodiment the tissue contacting surface 41 of tyne 18 is constructed of or coated with a non-conducting material, such as a suitable polymer. Surface 41 defines a cutting groove 43 through which blade 34 travels when it effects a cutting procedure.

As shown in Figure 4, tyne 20 is secured within housing segment 12a which preferably is detachable from housing segment 12b associated with tyne 18. Further, tyne 20 has a tissue-contacting surface 48 which faces first tyne 18. A central groove 52 is formed in surface 48, superimposable with cutting grooves 40 or 43 of tyne 18, to facilitate movement of the blade along longitudinal axis x.

In an embodiment in which surgical staples are to be deployed simultaneously with a cutting procedure, staple cartridge 38 is present within tyne 18. In addition, surface 48 of tyne 20 includes a mandrel with a plurality of staple-closing depressions 50 which correspond to the openings 42 in staple cartridge 38. Preferably, dual rows of depressions are disposed on either side of groove 52. In an embodiment in which a staple cartridge is not utilized, the surface 48 may be substantially smooth and absent depressions 50. In either embodiment, however, surface 48 of tyne 20 should be made of a conductive material so that it may serve as a return electrode for electrical energy delivered through the cutting blade.

In some instances, it may be desirable to apply electrosurgical energy through the surgical staples as well as through blade 34. One skilled in the art could easily modify the electrosurgical surgical tool described herein by connecting internal wire 28 to the staple ejection arms 45 as well as to the blade 34.

Figures 1 through 5 illustrate the connection of the cutting tool 10 to electrosurgical generator 26. As illustrated, an inner wire 28 extends between conductive bushing 51 and electrical connector 61 which protrudes from housing 12. Insulated wire 28 may be attached to electrical connector 61 through connector 63. Bushing 51 communicates electrical current from the generator 26 to blade 34, directly or through blade actuation arm 44. In a preferred embodiment arm 44 and blade 34 are able to slide within bushing 51 while maintaining electrical contact therewith.

In a preferred embodiment, the electrosurgical cutting tool 10 of the invention comprises a bipolar cutting tool in which the cutting blade 34 is electrically isolated from the remainder of the tool and serves as an electrode to deliver electrosurgical energy to the tissue. In this embodiment tyne 20 serves as the return or ground electrode.

The surface 48 of tyne 20 serves as a ground electrode. Accordingly, exterior ground wire 30 communicates with internal ground wire 33 through connector 31. Internal ground wire 33, in turn, is in electrical communication with a conductive internal anchoring component 19 of tyne 20.

As noted above, generator 26 supplies electrosurgical energy to the cutting blade. Virtually any generator which provides electrosurgical energy for medical applications may be used with the present invention. Preferably, the generator is a voltage determinative, low source impedance generator which provides radio frequency energy. Preferably, a suitable generator can supply up to 2 amps of current and has an impedance value of less than 10 ohms.

The energy supplied by generator 26 to the electrosurgical cutting device is preferably in the radio frequency range. Although virtually any frequency in the RF range may be supplied to the cutting device, the preferred frequency range is about 500 to 700 KHz, and most preferably about 550 KHz.

The energy requirements of the electrosurgical tool of the present invention are dynamic and depend to a great extent upon the impedance values of the tissue encountered by the blade during cutting procedures. The impedance of tissue varies among tissue types and the amount of blood present in or around the tissue. The amount of current delivered by the tool to the tissue is a function of the impedance of the tissue. Where tissue contacted has a lower impedance value, more current will be delivered to the tissue by the blade, and, conversely, less current will be delivered to tissue having a higher impedance value. Generally, the amount of current delivered to tissue ranges between about 0.5 and 2.0 amps. The voltage applied to the tissue between the blade and the return electrode typically is between about 50 to 100 volts rms.

The surgical tool of the present invention is particularly well adapted for use in surgical procedures which require transection of an organ such as the intestine. In operation, the tissue (e.g., intestine) is placed within space 22 defined by tynes 18 and 20. The blade is moved forward along the longitudinal axis x of tynes 18 and 20 by movement of lever 24. As the blade moves forward, it passes through the tissue causing it to be severed. Simultaneously, electrical energy (e.g., radio frequency energy), which may be activated for example by foot switch 32, is delivered to the tool. The electrosurgical current is communicated from the blade 34 to the tissue adjacent the blade and in the vicinity of the incision.

During a cutting procedure the blade should be actuated such that it requires approximately 1.5 to 4.5 seconds to move along its predetermined path to sever tissue. Current should be delivered through the blade to the tissue during the entire cutting procedure.

The application of electrical energy in this manner provides two advantages. Electrosurgical energy is delivered through the blade to adjacent tissue to allow for more effective cutting action, and to promote cauterization and/or tissue fusion which effectively eliminates all or substantially all bleeding which results from the incision. The cauterization and/or fusion effect imparted to tissue minimizes blood loss and increases the safety of the surgical procedure as cauterization occurs at substantially the same time that the incision is made.

In a preferred embodiment of the invention, the electrosurgical tool also includes a staple cartridge 38 which houses a supply of surgical staples to be supplied adjacent the incision. The staples may be deployed in one or more linear rows on either side of the incision to assist in closing the incision and sealing the severed end of the organ. The staples are deployed simultaneously with the cutting action of the blade and the tissue fusion effect imparted by the electrical energy.

One skilled in the art will appreciated that a variety of materials are well suited for the manufacture of the electrosurgical tool of this invention. For example, housing 12 and cartridge 38 may be made from or coated with various non-conducting polymers. The conductive components of the tool may be made of various metals, including surgical grade stainless steel and aluminum.

Although the invention is described with respect to the cutting tool illustrated in Figures 1 through 5, it is understood that various modifications may be made to the illustrated electrosurgical cutting device without departing from the scope of the invention. For example, a variety of blade actuation mechanisms may be used. Also, it is not necessary that tynes 18 and 20 take on the shape and orientation illustrated in the drawings. Moreover, the electrical connection between the generator may be made in ways other than those illustrated and described herein. Thus, the present invention is potentially applicable to virtually all electrosurgical cutting devices in which a cutting blade, moveable along a predetermined path, provides electrosurgical energy to incised tissue simultaneously with the cutting of tissue.

## Claims

1. An electrosurgical cutting device (10), comprising:
a housing (12) including a handle portion (14);
a cutting template element (16), adjacent to the handle portion (14), having substantially parallel first and second tyne elements (18, 20) which define a tissue engaging space (22) therebetween;
the first tyne element (18) having a pathway within the first tyne element (18) which defines a cutting path;
the second tyne element (20) having a tissue-contacting portion (48);
a moveable cutting blade (34);
means for moving the cutting blade (34) through the pathway to sever tissue;
**characterised in that**
the device further comprises selectively operable electrosurgical current delivery means (26) for communicating electrosurgical energy through the cutting blade (34) to tissue to cauterize tissue simultaneous with the cutting action of the blade (34);
the cutting blade (34) is electrically isolated from the tissue-contacting portion (48), said tissue-contacting portion being adapted to function as a return electrode.

2. A device (10) as claimed in Claim 1 wherein the first (18) and second (20) tyne elements are substantially parallel and define a tissue engaging space (22) through which, in use, the blade (34) moves to sever the tissue.

3. A device as claimed in Claim 1 or Claim 2 further comprising:
a cartridge (38) for housing a plurality of surgical staples (38), said cartridge (38) being disposed on the first tyne element (18), on a side thereof facing the second tyne element (20) and having a longitudinal groove (40) therein to accommodate passage of the cutting blade (34) ;
a means (45) for deploying the staples substantially simultaneously with the cutting action of the blade (34); and
a mandrel (50) for effecting closure of the staples, the mandrel (50) being disposed on a side of the second tyne element (20) facing the first tyne element (18).

4. A device (10) as claimed in Claim 3 wherein the cartridge (38) contains dual, linear rows of staple ejection ports (42) disposed on opposite sides of the groove (40), with surgical staples positioned to be ejected from the ports (42).

5. A device (10) as claimed in Claim 3 or Claim 4 wherein the second tyne element (20) further comprises a central groove (52) which cooperates with the longitudinal groove (40) of the cartridge (38).

6. A device (10) as claimed in any of Claims 1-5, wherein the current delivery means (26) comprises a conducting electrical wire (28) which communicates electrosurgical energy from a generator (26) to the cutting blade (34).

7. A device (10) as claimed in Claim 6 wherein the conducting electrical wire (28) communicates with the blade (34) through a conductive bushing (51) in electrical contact with the blade (34).

8. A device (10) as claimed in any of Claims 1-7 wherein the electrosurgical energy delivered by the device (10) is in the radio frequency range.

9. A device (10) as claimed in any of Claims 6-8 wherein the generator (26) is a voltage determinative, low impedance electrosurgical generator which supplies electrosurgical energy in the range of 500 to 700khz.

10. A device (10) as claimed in any of Claims 1-9 wherein the current of the electrosurgical energy delivered is in the range of about 0.5 to 2.0 amps.

11. A device (10) as claimed in any of Claims 1-10 wherein the voltage of the electrosurgical energy delivered is in the range of about 50 to 100 volts RMS.

## Patentansprüche

1. Elektrochirurgische Schneidvorrichtung (10), umfassend:
ein Gehäuse (12), das einen Griffteil (14) einschließt;
ein Schneidschablonenelement (16) angrenzend an den Griffteil (14), das im wesentlichen parallele erste und zweite Zinkenelemente (18, 20) aufweist, die einen Gewebeangriffsraum (22) zwischen sich definieren;
wobei das erste Zinkenelement (18) einen Weg innerhalb des ersten Zinkenelements (18) aufweist, der einen Schneidweg begrenzt;
und das zweite Zinkenelement (20) einen gewebeberührenden Teil (48) aufweist;
eine bewegliche Schneidklinge (34);
Mittel zum Bewegen der Schneidklinge (34) durch den Weg zum Auftrennen von Gewebe;
**dadurch gekennzeichnet, dass**
die Vorrichtung (10) weiter selektiv bedienbare elektrochirurgische Stromzuführmittel (26) zum Übermitteln elektrochirurgischer Energie durch die Schneidklinge (34) zum Gewebe aufweist, um Gewebe gleichzeitig mit der Schneidtätigkeit der Klinge (34) abzuätzen;
wobei die Schneidklinge (34) elektrisch von dem gewebeberührenden Teil (48) isoliert ist, und der gewebeberührende Teil ausgelegt ist, um als eine Rückelektrode zu wirken.

2. Vorrichtung (10) nach Anspruch 1, bei der das erste (18) und zweite (20) Zinkenelement im wesentlichen parallel sind und einen gewebeergreifenden Raum (22) begrenzen, durch den sich die Klinge (34) in Gebrauch zum Auftrennen des Gewebes bewegt.

3. Vorrichtung nach Anspruch 1 oder Anspruch 2, weiter umfassend:
eine Patrone (38) zum Unterbringen einer Anzahl chirurgischer Heftklammern (38), wobei die genannte Patrone (38) an dem ersten Zinkenelement (18) auf einer Seite desselben angeordnet ist, die zu dem zweiten Zinkenelement (20) gerichtet ist und eine Längsnut (40) darin zum Aufnehmen des Durchgangs der Schneidklinge (34) aufweist;
ein Mittel (45) zum Auseinanderziehen der Heftklammern im wesentlichen gleichzeitig mit der Schneidtätigkeit der Klinge (34); und
eine Spindel (50) zum Bewirken von Verschluss der Heftklammern, wobei die Spindel (50) auf einer Seite des zweiten Zinkenelements (20) angeordnet ist, die zu dem ersten Zinkenelement (18) gerichtet ist.

4. Vorrichtung (10) nach Anspruch 3, bei der die Patrone (38) doppelte, lineare Reihen von Heftklammerausstoßöffnungen (42) angeordnet auf gegenüberliegenden Seiten der Nut (40) enthält, wobei chirurgische Heftklammern positioniert sind, um aus den Öffnungen (42) ausgestoßen zu werden.

5. Vorrichtung (10) nach Anspruch 3 oder Anspruch 4, bei der das zweite Zinkenelement (20) weiter eine mittige Nut (52) aufweist, die mit der Längsnut (40) der Patrone (38) zusammenwirkt.

6. Vorrichtung (10) nach einem der Ansprüche 1-5, bei der das Stromzuführmittel (26) einen leitenden elektrischen Draht (28) aufweist, der elektrochirurgische Energie von einem Generator (26) zu der Schneidklinge (34) übermittelt.

7. Vorrichtung (10) nach Anspruch 6, bei der der leitende elektrische Draht (28) mit der Klinge (34) durch eine leitende Buchse (51) in elektrischem Kontakt mit der Klinge (34) kommuniziert.

8. Vorrichtung (10) nach einem der Ansprüche 1-7, bei der die durch die Vorrichtung (10) zugeführte elektrochirurgische Energie im Funkfrequenzbereich liegt.

9. Vorrichtung (10) nach einem der Ansprüche 6 -8, bei der der Generator (26) ein spannungsbestimmender, niederohmiger elektrochirurgischer Generator ist, der elektrochirurgische Energie im Bereich von 500 bis 700 kHz zuführt.

10. Vorrichtung (10) nach einem der Ansprüche 1-9, bei der der Strom der zugeführten elektrochirurgischen Energie im Bereich von etwa 0,5 bis 2,0 Ampere liegt.

11. Vorrichtung (10) nach einem der Ansprüche 1-10, bei der die Spannung der zugeführten elektrochirurgischen Energie im Bereich von etwa 50 bis 100 Volt Effektivwert liegt.

## Revendications

1. Dispositif électrochirurgical coupant (10) comprenant :
un logement (12) comportant une section poignée (14) ;
un élément gabarit de coupe (16), qui est adjacent à la section poignée (14), ayant un premier et un deuxième éléments dents (18, 20) essentiellement parallèles qui définissent entre eux un espace (22) d'engagement du tissu ;
le premier élément dent (18) ayant un trajet à l'intérieur du premier élément dent (18) qui définit un trajet de coupe ;
le deuxième élément dent (20) ayant une section (48) en contact avec le tissu ;
une lame de coupe mobile (34) ;
un moyen permettant de déplacer la lame de coupe (34) par le trajet afin de sectionner le tissu;
**caractérisé en ce que**
le dispositif comporte en outre un moyen (26) d'alimentation de courant électrochirurgical capable de fonctionner de manière sélective permettant d'acheminer de l'énergie électrochirurgicale à travers la lame de coupe (34) jusqu'au tissu pour que le tissu puisse être cautérisé simultanément à l'action de coupe de la lame (34);
la lame de coupe (34) est isolée sur le plan électrique de la section (48) en contact avec le tissu, ladite section en contact avec le tissu étant adaptée de façon à jouer le rôle d'électrode de retour.

2. Dispositif (10), selon la revendication 1, dans lequel le premier (18) et le deuxième (20) éléments dents sont essentiellement parallèles et définissent entre eux un espace (22) d'engagement du tissu à travers lequel, au moment de l'utilisation, la lame (34) se déplace pour sectionner le tissu.

3. Dispositif, selon la revendication 1 ou la revendication 2, comprenant en outre :
une cartouche (38) destinée à abriter une pluralité d'agrafes chirurgicales (38), ladite cartouche (38) étant disposée sur le premier élément dent (18), sur un côté de celui-ci faisant face au deuxième élément dent (20) et possédant dans celle-ci une rainure longitudinale (40) afin de permettre le passage de la lame de coupe (34) ;
un moyen (45) permettant de déployer les agrafes essentiellement en simultanéité avec l'action de coupe de la lame (34); et
un mandrin (50) permettant d'effectuer la fermeture des agrafes, le mandrin (50) étant disposé sur l'une des faces du deuxième élément dent (20) faisant face au premier élément dent (18).

4. Dispositif (10), selon la revendication 3, dans lequel la cartouche (38) présente des doubles rangées linéaires d'orifices (42) d'éjection d'agrafes qui sont disposées sur les côtés opposés de la rainure (40), alors que les agrafes chirurgicales sont positionnées pour être éjectées hors des orifices (42).

5. Dispositif (10), selon la revendication 3 ou la revendication 4, dans lequel le deuxième élément dent (20) comprend en outre une rainure centrale (52) qui agit en interaction avec la rainure longitudinale (40) de la cartouche (38).

6. Dispositif (10), selon l'une quelconque des revendications 1-5, dans lequel le moyen (26) d'alimentation de courant comprend un fil (28) électriquement conducteur qui transmet l'énergie électrochirurgicale d'une génératrice (26) à la lame de coupe (34).

7. Dispositif (10), selon la revendication 6, dans lequel le fil (28) électriquement conducteur communique avec la lame (34) par l'intermédiaire d'une douille conductrice (51) qui est en contact électrique avec la lame (34).

8. Dispositif (10), selon l'une quelconque des revendications 1-7, dans lequel l'énergie électrochirurgicale fournie par le dispositif (10) se trouve dans la gamme des radiofréquences.

9. Dispositif (10), selon l'une quelconque des revendications 6-8, dans lequel la génératrice (26) est une génératrice électrochirurgicale de faible impédance et à détermination de tension qui fournit de l'énergie électrochirurgicale dans la gamme des 500 à 700 kHz.

10. Dispositif (10), selon l'une quelconque des revendications 1-9, dans lequel le courant de l'énergie électrochirurgicale fournie se situe dans la gamme allant de 0,5 A à 2,0 A environ.

11. Dispositif (10), selon l'une quelconque des revendications 1-10, dans lequel la tension de l'énergie électrochirurgicale fournie se situe dans la gamme allant de 50 à 100 Volts environ (volts efficaces).
